(19) Europäisches Patentamt
European Patent Office
Office européen des brevets

(11) Veröffentlichungsnummer : **0 119 226**
**B1**

(12) # EUROPÄISCHE PATENTSCHRIFT

(45) Veröffentlichungstag der Patentschrift : **29.07.87**

(51) Int. Cl.⁴ : **A 23 L 3/34**, A 23 L 3/36, A 23 B 4/08, A 23 B 4/14, A 23 K 3/00, A 01 N 43/80, A 01 N 33/12

(21) Anmeldenummer : **83902888.3**

(22) Anmeldetag : **08.09.83**

(86) Internationale Anmeldenummer : **PCT/EP 83/00234**

(87) Internationale Veröffentlichungsnummer : **WO/8400877 (15.03.84 Gazette 84/07)**

(54) **VERWENDUNG EINES MITTELS ZUR ANTIMIKROBIELLEN BEHANDLUNG VON LEBENS- UND FUTTERMITTELN.**

(30) Priorität : **10.09.82 DE 3233607**

(43) Veröffentlichungstag der Anmeldung : **26.09.84 Patentblatt 84/39**

(45) Bekanntmachung des Hinweises auf die Patenterteilung : **29.07.87 Patentblatt 87/31**

(84) Benannte Vertragsstaaten : **AT BE CH DE FR GB LI LU NL SE**

(56) Entgegenhaltungen :
**CA-A- 1 131 404**
**DE-A- 2 438 035**
**DE-A- 2 450 666**
**DE-A- 2 930 865**
**GB-A- 2 011 790**
**GB-A- 2 044 776**
**US-A- 3 761 488**
**US-A- 4 173 643**

(73) Patentinhaber : **RIEMER, Freimut**
**Höfgensweg 8**
**D-5135 Selfkant-Tüddern (DE)**

(72) Erfinder : **RIEMER, Freimut**
**Höfgensweg 8**
**D-5135 Selfkant-Tüddern (DE)**

(74) Vertreter : **Paul, Dieter-Alfred, Dipl.-Ing.**
**Fichtestrasse 18**
**D-4040 Neuss 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 119 226 B1

**Beschreibung**

Die Erfindung betrifft die Verwendung eines Gemisches aus einer quaternären Ammoniumverbindung mit einer Isothiazolinone-Verbindung.

In der DE-A-24 50 660 ist ein Verfahren zur antimikrobiellen Behandlung von bestimmten Futtermitteln beschrieben, bei dem quaternäre Ammoniumverbindungen zusammen mit Polyalkylenglykolen in wässriger Lösung bzw. Dispersion dem Futtermittel beigemischt und bei erhöhter Temperatur auf diese zur Anwendung gebracht werden. Mit diesem Verfahren können insbesondere Trockenfuttermehle mit befriedigendem Erfolg antimikrobiell behandelt werden. Das dabei verwendete Mittel zeichnet sich durch eine relativ gute Depotwirkung und geringe Toxizität sowie durch Preiswürdigkeit aus.

Allerdings hat sich sein Wirkungsspektrum gegen die verschiedenen Mikroorganismen als nicht so breit erwiesen, wie es im Hinblick auf eine möglichst universelle Anwendbarkeit wünschenswert wäre. Auch die Depotwirkung befriedigte bisher nicht völlig. Dies gilt vor allem für die antimikrobielle Behandlung von in der DE-PS 24 50 666 nicht angegebenen Futtermitteln sowie von Lebensmitteln, insbesondere von zur Tiefkühlung vorgesehenen Lebensmitteln.

Gerade hier besteht jedoch wegen des weltweit ansteigenden Bedarfs an Tiefkühl-Lebensmitteln sowie deren Transport übert sehr lange Transportwege eine sehr hohe Gefahr eines möglichen Salmonellenbefalls. Dieser Befall kann bereits während der Verarbeitungsstufe oder durch kurzzeitigen Temperaturanstieg über 0 °C während der Transportphase eintreten. Die Entwicklung pathogener Keime, speziell von Salmonellen, ist dann evident. Tagtäglich werden weltweit Fälle von Salmonellose mit todesfolge festgestellt.

Eine antimikrobielle Behandlung von Tiefkühl-Lebensmitteln ist deshalb angezeigt, um die Waren vor dem Einfrierprozeß von pathogenen Keimen freizuhalten. Weiterhin sollte eine gute Depotwirkung erzielt werden, so daß im Produkt bzw. im Auftauwasser keine pathogenen Keime, insbesondere Salmonellen, auftreten können. Physikalische Keimtötungsverfahren (Hitzesterilisation, UV-Bestrahlung) lassen sich nicht oder nur im beschränkten Umfang anwenden. Die Hitzesterilisation ist wegen des Produktionsablaufes bei der Tiefkühl-Herstellung nicht möglich. Die immensen Energiekosten, die eine schnelle Abkühlung auf die geforderten Temperaturen mit sich bringen würden, zusammen mit der Gefahr einer zwischenzeitlich erneuten Infektion der Waren ist ohne weiteres erkennbar.

Ein weiterer Nachteil des aus der DE-PS 24 50 666 bekannten Mittels besteht darin, daß die Lösung bzw. Dispersion zur Verbesserung der Löslichkeit und Wirksamkeit bei der Anwendung erwärmt werden muß. Auch dies schränkt den Anwendungsbereich — abgesehen vom apparativen und energiemäßigen Aufwand — ein.

In der US-A-4 173 643 ist eine Mischung einer Isothiazolinone-Verbindung mit einem quaternären Ammoniumsalz beschrieben. Nach Spalte 4, insbesondere den zeilen 43 bis 53, soll diese Mischung als reines Desinfektionsmittel verwendet werden, mit dem Oberflächen, die mit organischen Materialien verschmutzt sind, behandelt werden sollen. An die Behandlung von Lebens- und Futtermitteln — sie stellen ebenfalls organisch Materialien dar — zu deren Konservierung ist nicht gedacht.

In der GB-A-2 011 790 wird auf die Aggressivität des von Rohm and Haas Co. entwickelten Mittels hingewiesen. Zur Desinfektion und Konservierung verschiedener, industriell angewendeter Stoffe wird die Kombination einer Isothiazolinone-Verbindung mit Dimethylolharnstoff, Formaldehyd und aliphatischen Glykolen vorgeschlagen. Auch hier ist an die Konservierung von zum Verzehr bestimmten Mitteln nicht gedacht. Vielmehr wird in der nachfolgenden GB-A-2 044 776 zwar darauf hingewiesen, daß sich die aus der GB-A-2 011 790 bekannte Wirkstoffkombination auch für die Desinfizierung von Verpackungsmaterial für Futtermittel eignet, wobei jedoch soweit wie möglich vermieden werden soll, daß die Wirkstoffkombination von der Verpackung in das Futtermittel eindringt, und zwar unter Hinweis auf die Giftigkeit dieser Wirkstoffkombination.

In der DE-A-29 30 865 wird als Desinfektions- und Konservierungsmittel die Kombination einer polymeren quaternären Ammoniumverbindung mit einer 3-Isothiazolon-Verbindung vorgeschlagen, wobei als Anwendungsgebiet die Desinfektion von Wäschekammern für die Luftbefeuchtung von Klimaanlagen und von Wasserkreisläufen, insbesondere Kühlwasserkreisläufen, angegeben wird. Auch in dieser Druckschrift fehlt jeder Hinweis auf die Konservierung zum Verzehr bestimmter Lebens- und Futtermittel. Entsprechendes gilt für die DE-A-24 38 035, in der ein Gemisch aus einer polymeren quaternären Ammoniumverbindung mit einer bestimmten Isothiazolinone-Verbindung beschrieben ist, wobei als Anwendungsgebiet wässrige Medien, beispielsweise Wasser/Oel-Emulsionen, bei der Lagerung von Anstrichfarben und Klebstoffen auf Wasserbasis und in Kühlwassersystemen genannt werden. Die Konservierung von Lebens- und Futtermitteln ist hier gleichfalls nicht angegeben.

Die Erfindung liegt die Aufgabe zugrunde, ein Mittel bereitzustellen, mit dem eine Vielzahl von Futter- und Lebensmitteln, insbesondere von Tiefkühl-Lebensmitteln antimicrobiell behandelt und konserviert werden kann und das sich durch ein sehr breites Wirkungsspektrum und gute Depotwirkung auszeichnet.

Diese Aufgabe wird erfindungsgemäß dadurch gelöst, daß für den vorgenannten Verwendungszweck ein Gemisch aus einer quaternären Ammoniumverbindung mit einer Isothiazolinone-Verbindung mit einem Gewichts-Mischungsverhältnis der beiden Verdindungen im Bereich 1 : 1 bis 20 : 1 genommen

wird, wobei die Isothiazolinone-Verbindung(en) die allgemeine Formel hat bzw. haben :

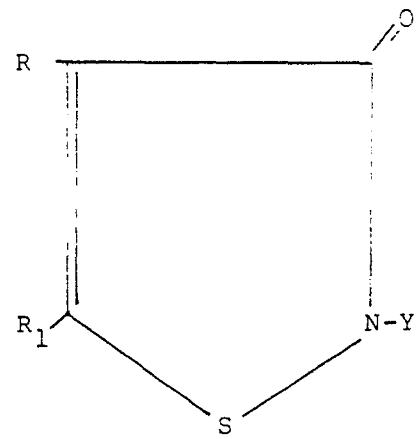

bei der sowohl R als auch $R_1$ Wasserstoffatome, Chlor-atome, Bromatome oder Methylgruppen bedeuten und bei der Y eine Alkylgruppe mit drei bis achtzehn Kohlenstoffatomen, eine Cyklohexylgruppe, eine gegebenenfalls durch eine niedermolekulare Alkylgruppe oder niedermolekulare Alkoxygruppe substituierte Aralkylgruppe mit bis zu zehn Kohlenstoffatomen, eine 2- oder 4-Chlorbenzylgruppe, eine 2,4- oder 3,4-Dichlorbenzylgruppe, eine Hydroxymethylgruppe, ein gegebenenfalls durch Chlor, niedermolekulare Alkylgruppen, eine Nitrogruppe oder Carbäthoxygruppe substituierter Phenylrest oder eine Carbamoxyalkylgruppe der allgemeinen Formel :

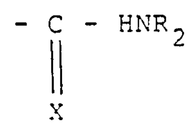

bedeutet, worin X ein Sauerstoffatom oder Schwefelatom und $R_2$ eine Alkylgruppe mit einem bis acht Kohlenstoffatomen, eine Carbäthoxymethylgruppe, eine Chlorphenylgruppe, eine 2,5- oder 3,4-Dichlorphenylgruppe oder eine 4-Nitrophenylgruppe bedeutet.

Gegenüber dem aus der DE-A-24 50 666 bekannten Konservierungsmittel zeichnet sich das erfindungsgemäße Konservierungsmittel durch eine wesentlich verbesserte antimikrobielle Wirkung und durch ein breiteres Wirkungsspektrum aus. Es ist deshalb für eine Vielzahl von Futtermitteln, beispielsweise Trockenfutter aus Fisch- und Walabfällen, Garnelenmehlen, Tierkörpermehlen, Fleischfuttermehlen und Blutmehlen, einsetzbar, aber auch für Lebensmittel, wie beispielsweise Fisch, Fleisch, Garnelen, Geflügel oder dergleichen, insbesondere wenn sie zur Tiekühlung bestimmt sind. Dabei hat es sich gezeigt, daß eine ausgezeichnete Depotwirkung über sehr lange Zeit erreicht wird, keine Verfärbungen und Geruchsveränderungen auftreten und es zudem biologisch abbaubar ist. Es ist schon in sehr geringen Konzentrationen hochwirksam, und zwar auch schon wenige Grade über 0 °C. Auf eine Erwärmung, wie dies bei dem Mittel nach der DE-A-24 50 666 noch erforderlich war, kann hier verzichtet werden. Dabei ist auffallend die an sich nicht zu erwartende selektive Wirkung mit der Folge, daß das zu behandelnde Lebens- und Futtermittel in allen Eigenschaften nicht beeinträchtigt wird, andererseits die Konservierungswirkung gegenüber mikrobiellen Befall äußerst wirksam ist.

Keines der auf dem Markt befindlichen physikalischen Keimtötungsverfahren kann mit ähnlich günstigen Eigenschaften aufwarten. Entweder sind sie aus verfahrenstechnischer Sicht oder sogar hinsichtlich der Kosten und der Wirkung, insbesondere der Depotwirkung, dem erfindungsgemäßen Konservierungsmittel wesentlich unterlegen.

Diese eindrucksvollen Vorteile waren nicht zu erwarten, denn die quaternären Ammoniumverbindungen haben kein so breites antimikrobielles Wirkungsspektrum und eine vergleichsweise kurze Depotwirkung. Zudem verlieren sich bei Anwesenheit von anionenaktiven Substanzen und eiweißhaltiger Begleitstoffe ihre keimtötende Wirkung. Den Isothiazolinone-Verbindungen allein, wie sie aus der US-A-3 761 488 bekannt sind, fehlt es an schneller Abtötungswirkung gegenüber Mikroorganismen, wie Pilzen, Bakterien, Hefen und Algen, was jedoch ein wesentliches Erfordernis für die Behandlung in Wasch-, Spül-, Tauch-, Sprüh- oder Tränkverfahren ist.

Sehr günstige Werte werden mit Isothiazolinone-Verbindungen erzielt, die als Isothiazolin-Verbindungen mit der Zusammensetzung 5-chlor-2-methyl-4-isothiazolin-3-one und 2-methyl-4-isothiazolin-3-one im Gewichtsverhältnis 3 : 1 ausgebildet sind. Die zugehörige grafische Darstellung der Formel ist dem Anspruch 2 zu entnehmen. Die Molekulargewichte dieser Isothiazolin-Verbindungen betragen dabei ca. 148,5 bzw. 114.

Das Gewichts-Mischungsverhältnis der quaternären Ammoniumverbindung zu der Isothiazolinone-Verbindung sollte vorzugsweise im Bereich von 9 : 1 liegen.

Bei der Anwendung für Futtermittel sollte ein Gewichtsverhältnis von einem Teil des erfindungsgemässen Mittels auf 50 bis 250 Teile des Futtermittels eingehalten werden. Soweit das Mittel für Lebensmittel angewendet wird, empfiehlt sich eine wässrige Lösung in einer Konzentration von 1 : 500 bis 1 : 2 500, die beispielsweise als Wasch-, Spül-, Sprüh- oder Tränkwasser im letzten Produktionsschritt vor der Tiefkühlphase verwendet werden kann.

Die Herstellung des vorbeschriebenen Mittels geschieht durch Vermischung der jeweiligen Isothiazolinone-Verbindung mit der jeweiligen quaternären Ammoniumverbindung, wobei zweckmäßigerweise erstere der letzteren zugemischt wird. Vorzugsweise soll die Vermischung bei Temperaturen zwischen 25 bis 45 °C, ansonsten bei Umgebungsdruck stattfinden.

Die Erfindung ist im Nachstehenden beispielsweise erläutert.

Beispiel 1

Es wurde ein Gemisch gebildet mit neun Gewichtsteilen eines quaternären Ammoniumsalzes mit den Substituenten Dimethyl, Benzyl und n-Alkyl, letzteres in der Verteilung 40 % C12, 50 % C14 und 10 % C16, und ein Gewichtsteil einer Isothiazolin-Verbindung in der Zusammensetzung 5-chloro-2-methyl-4-isothiazolin-3-one (3) + 2-methyl-4-isothiazolin-3-one (1). Dieses Gemisch wurde im Verhältnis von 1 : 100 einer Fischmehl-bzw. Tiermehl-Rohmenge zugesetzt. Die Rohmengen wurden mit verschiedenen Salmonellastämmen (Salmonella Eimsbüttel, S. Livingstone, S. Montevideo) beimpft, mit Tetrathionate enrichment Medium ganz überschichtet und bei 40 °C bebrütet. 24 Stunden später wurde auf selektives Media (S. S. Agar, Wilson & Blair, McConckey) übergeimpft und auf Salmonellen-Wachstum überprüft. Einige der Versuchsmuster wurden während der Versuchslaufzeit reinfiziert, um die Resistenz gegenüber erneutem Salmonellenbefall festzustellen.

Während des ersten Versuchszeitraums über drei Monate wurde sowohl mit als auch ohne Reinfektion kein erneuter Salmonellenbefall festgestellt, und zwar weder im Fisch- noch im Tiermehl. Die Muster wurden nach sechs, acht und zwölf Monaten erneut untersucht, um festzustellen, ob nach dieser Lagerzeit die am Anfang beimpften Salmonellenstämme wieder isoliert werden können. Das Ergebnis war bei allen Muster negativ. Demnach ergibt das in diesem beispiel verwendete Gemisch einen sofortigen Schutz gegen Salmonellenbefall mit einer Depotwirkung von mindest zwölf Monaten bei handelsüblicher Lagerung.

Beispiel 2

Es wurde dasselbe Gemisch wie im Beispiel 1 im Verhältnis 1 : 1 000 dem Wasch-, Spül-, Sprüh- oder Tränkwasser zur Behandlung von für die Tiefkühlung vorgesehenen Lebensmitteln zugegeben, und zwar in dem jeweils letzten Produktionsschritt vor der Tiefkühlphase. Die anschließend schockgefrorenen Produkte wurden gemäß den Herstellervorschriften gelagert und nach dem Auftauen einer bakteriologischen Prüfung auf Salmonellenbefall unterzogen. Weder nach kurzen noch nach längeren Tiefkühllagerzeiten wurden im Produkt oder im Auftauwasser Salmonellen oder andere pathogene Keime festgestellt. Ein Versuch, in dem einer kurzzeitigen Auftauperiode auf + 5 °C eine erneute Tiefkühlperiode folgte, zeigte nach dem entgültigen Auftauen des Produkts ebenfalls einen negativen Befund.

Beispiel 3

Zur Darstellung der bakteriziden und fungiziden Wirkung wurde ein üblicher Test durchgeführt, bei dem die Wirksamkeit von zwei Testkomponenten untersucht wurde. Die eine testkomponente war das schon aus den Beispielen 1 und 2 bekannte Gemisch, die andere, hier Vergleichskomponente genannt, das in der DE-PS 24 50 666 beispielhaft erwähnte Gemisch. Bei diesem Gemisch wurde ein Gewichtsteil eines quaternären Ammoniumsalzes mit den Substituenten Dimethyl, Äthyl, Benzyl und n-Alkyl, letzteres in der Verteilung 50 % C12, 30 % C14, 17 % C16 und 3 % C18, mit zwei Gewichtsteilen einer wässrigen Polyäthylenglykol-Dispersion versetzt, wobei sich ein Feststoffgehalt von 20 % und ein Molekulargewicht von ca. 600 ergab.

Test- und Vergleichskomponenten wurden jeweils in Tripticase Sojabohnenextrakt seriell verdünnt. Sie wurden dann einer 1 : 100 Beimpfung mit Testbakterien, und zwar 24 Stunden alten Bakterienkulturen, und einer Pilzspurensuspension, und zwar sieben bis 14 Tage alten und in 7 ml deionisiertem Wasser gewaschenen Pilzkulturen, unterworfen. Der bakterizide bzw. fungizide Wirkungswert wurde bei Bakterien nach 24 Stunden Bebrütungszeit bei 37 °C und bei Pilzen nach 48 Stunden Bebrütungszeit bei 28 °C bis 30 °C visuell bestimmt. Dabei ergaben sich die in der nachfolgenden Tabelle angegebenen Werte in ppm der Aktivsubstanzen :

| Mikroorganismus | Testkomponente | Vergleichskomponente |
|---|---|---|
| Aspergillus niger | 500 | 1000 |

(Fortsetzung)

| Mikroorganismus | Testkomponente | Vergleichskomponente |
|---|---|---|
| Pseudomonas aeruginosa | 25 | 7o |
| Salmonella typhosa | 9 | 2o |
| Staphhylococcus aureus | 7 | 2o |
| Escherichia coli | 1o | 25 |

Die Tabelle zeigt, daß die Wirkungswerte der erfindungsgemäßen Testkomponente im Vergleich zu der aus der DE-PS 24 50 666 bekannten Vergleichskomponente, bezogen auf die jeweilige Aktivsubstanz, hinsichtlich aller hier genannten Mikroorganismen wesentlich verbessert ist.

**Patentansprüche**

1. Verwendung eines Gemisches aus einer quaternären Ammoniumverbindung mit einer Isothiazolinone-Verbindung mit einem Gewichts-Mischungsverhältnis der beiden Verbindungen im Bereich 1 : 1 bis 20 : 1 als Konservierungsmittel für insbesondere zur Tiefkühlung vorgesehene Lebensmittel sowie für Futtermittel, wobei die Isothiazolinone-Verbindung(en) die folgende allgemeine Formel hat bzw. haben :

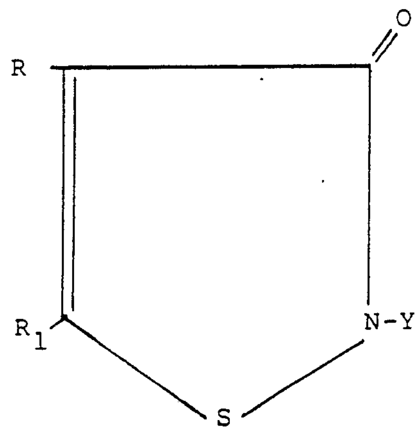

bei der sowohl R als auch $R_1$ Wasserstoffatome, Chlor-atome, Bromatome oder Methylgruppen bedeuten und bei der Y eine Alkylgruppe mit drei bis achtzehn Kohlenstoffatomen, eine Cyclohexylgruppe, eine gegebenenfalls durch eine niedermolekular Alkylgruppe oder niedermolekulare Alkoxygruppe substituierte Aralkylgruppe mit bis zu zehn Kohlenstoffatomen, eine 2- oder 4-Chlorbenzylgruppe, eine 2,4- oder 3,4-Dichlorbenzylgruppe, eine Hydroxymethylgruppe, ein gegebenenfalls durch Chlor, niedermolekulare Alkylgruppen, eine Nitrogruppe oder Carbäthoxygruppe substituierter Phenylrest oder eine Carbamoxyalkylgruppe der allgemeinen Formel :

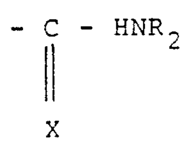

bedeutet, worin X ein Sauerstoffatom oder Schwefelatom und $R_2$ eine Alkylgruppe mit einem bis acht Kohlenstoffatomen, eine Carbäthoxymethylgruppe, eine Chlorphenylgruppe, eine 2,5- oder 3,4-Dichlorphenylgruppe oder eine 4-Nitrophenylgruppe bedeutet.

2. Verwendung nach Anspruch 1, dadurch gekennzeichnet, daß die Isothiazolinone-Verbindung(en) eine isothiazolin-Verbindung mit der Zusammensetzung 5-chloro-2-methyl-4-isothiazolin-4-one und 2-methyl-4-isothiazolin-3-one im Gewichtsverhältnis 3 : 1 ist bzw. sind und die Formel hat bzw. haben :

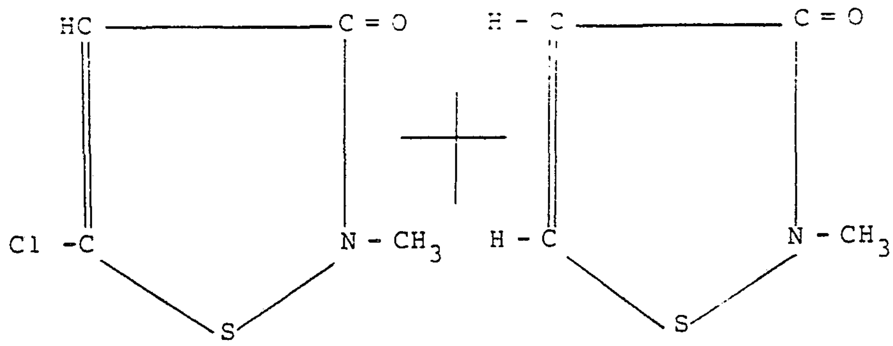

3. Verwendung nach Anspruch 1, dadurch gekennzeichnet, daß die quaternäre Ammoniumverbindung die Formel

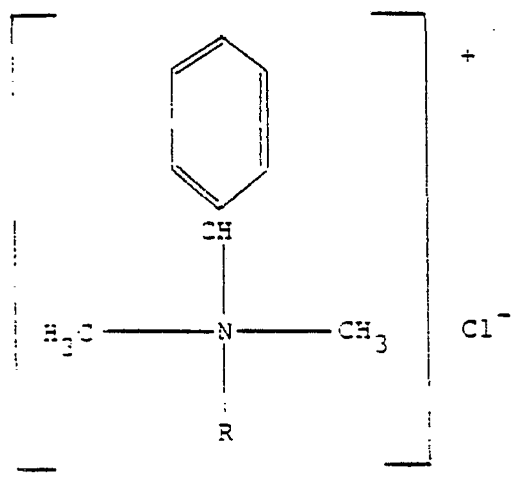

bei der der Substituent R ein besitzt, $C_{12}$-$C_{16}$ n-Alkyl ist.

4. Verwendung nach Anspruch 3, dadurch gekennzeichnet, daß das n-Alkyl die Verteilung 40 % $C_{12}$, 50 % $C_{14}$ und 10 % $C_{16}$ hat.

5. Verwendung nach Anspruch 1 oder 2, dadurch gekennzeichnet, daß das Gewichts-Mischungsverhältnis im Bereich von 9 : 1 liegt.

6. Verwendung des Mittels nach einem der Ansprüche 1 bis 3 für Futtermittel in einem Gewichtsverhältnis von einem Teil des Mittels auf 50 bis 250 Teile des Futtermittels.

7. Verwendung des Mittels nach einem der Ansprüche 1 bis 3 für Lebensmittel in einer wässrigen Lösung in einer Konzentration von 1 : 500 bis 1 : 2 500.

**Claims**

1. Use of a mixture of a quaternary ammonium compound with an isothiazolinone compound with a mixture ratio by weight of the two compounds in the range 1 : 1 to 20 : 1 as a preservative for food products intended especially for low-temperature cooling, and for fodder products, the isothiazolinone compound(s) having the following general formula :

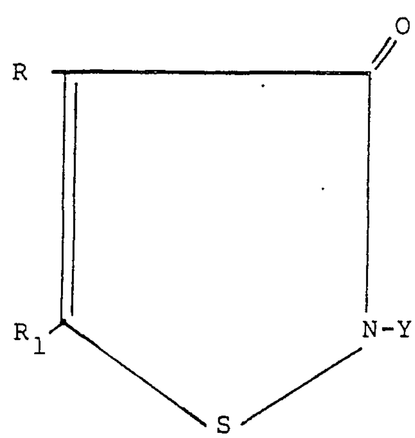

wherein both R and R1 signify hydrogen atoms, chlorine atoms, bromine atoms or methyl groups, and wherein Y signifies an alkyl group with three to eighteen carbon atoms, a cyclohexyl group, and aralkyl group substituted possibly by a low-molecular alkyl group of low-molecular alkoxy group and with up to ten carbon atoms, a 2- or 4-chlorobenzyl group, a 2,4- or 3,4-dichlorobenzyl group, a hydroxymethyl group, a phenyl group possibly substituted by chlorine, low-molecular alkyl groups, a nitro group or carbethoxy group, or a carbamoxyalkyl group of the general formula :

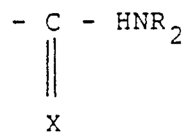

wherein X signifies an oxygen atom or sulphur atom and $R_2$ an alkyl group with one to eight carbon atoms, a carbethoxymethyl group, a chlorophenyl group, a 2,5- or 3,4-dichlorophenyl group, or a 4-nitrophenyl group.

2. Use according to claim 1, characterised in that the isothiazolinone compound(s) is(are) an isothiazoline compound with the composition 5-chloro-2-methyl 4-isothiazolin-4-one and 2-methyl 4-isothiazolin-3-one in the ratio by weight 3 : 1, and has(have) the formula :

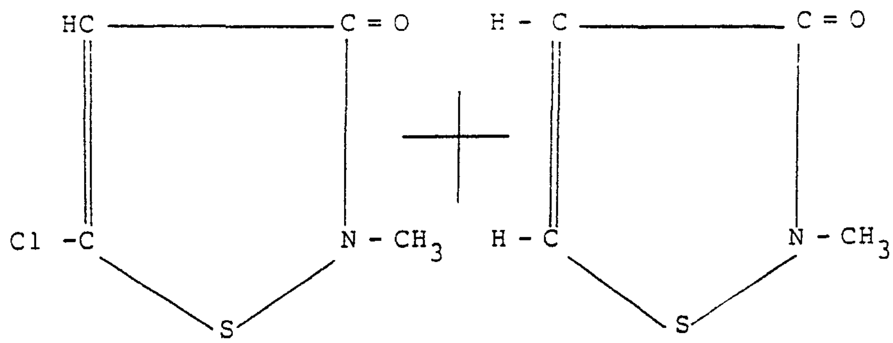

3. Use according to claim 1, characterised in that the quaternary ammonium compound has the formula

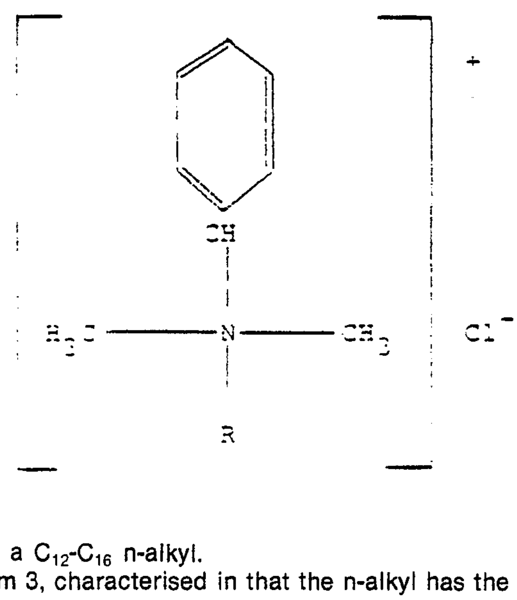

wherein the substituent R is a $C_{12}$-$C_{16}$ n-alkyl.

4. Use according to claim 3, characterised in that the n-alkyl has the distribution 40 % $C_{12}$, 50 % $C_{14}$ and 10 % $C_{16}$.

5. Use according to claim 1 or 2, characterised in that the mixture ratio by weight is in the region of 9 : 1.

6. Use of the combination according to one of claims 1 to 3 for fodder products in a weight ratio of one part of the combination to 50 to 250 parts of the fodder product.

7. Use of the combination according to one of claims 1 to 3 for food products in an aqueous solution in a concentration of 1 : 500 to 1 : 2 500.

**Revendications**

1. Utilisation d'une composition constituée d'un composé d'ammonium quaternaire et d'un composé d'isothiazolinone, en un rapport pondéral des deux composés de l'ordre de 1 : 1 à 20 : 1, comme conservateur de denrées alimentaires destinées notamment à être surgelées, ainsi que de produits fourragers, le(les) composé(s) d'isothiazolinone ayant la formule développée suivante :

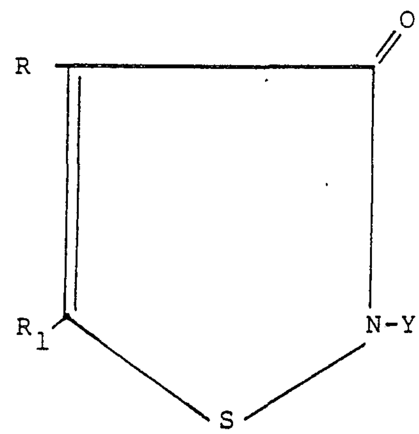

dans laquelle aussi bien R que $R_1$ représentent des atomes d'hydrogène, des atomes de chlore, des atomes de brome ou des groupes méthyle, et dans laquelle Y représente un groupe alcoyle ayant de trois à dix-huit atomes de carbone, un groupe cyclohexyle, un groupe aralcoyle ayant jusqu'à dix atomes de carbone, substitué éventuellement par un groupe alcoyle à bas poids moléculaire ou par un groupe alcoxy à bas poids moléculaire, un groupe 2-chlorobenzyle ou 4-chlorobenzyle, un groupe 2,4-dichlorobenzyle ou 3,4-dichlorobenzyle, un groupe hydroxy méthyle, un radical phényle éventuellement substitué par du chlore, par des groupes alcoyle à bas poids moléculaire, par un groupe nitro ou par un groupe carbéthoxy ou un groupe carbamoxyalcoyle de formule développée :

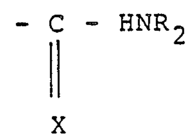

dans laquelle X représente un atome d'oxygène ou un atome de soufre et $R_2$ un groupe alcoyle ayant de un à huit atomes de carbone, un groupe carbéthoxyméthyle, un groupe chlorophényle, un groupe 2,5-dichlorophényle ou 3,4-dichlorophényle ou un groupe 4-nitrophényle.

2. Utilisation suivant la revendication 1, caractérisée en ce que le(les) composé(s) d'isothiazolinone est(sont) un(des) composé(s) d'isothiazoline comprenant de la 5-chloro-2-méthyl-4-isothiazoline-4-one et de la 2-méthyl-4-isothazoline-3-one en le rapport pondéral de 3 : 1 et a(ont) la formule :

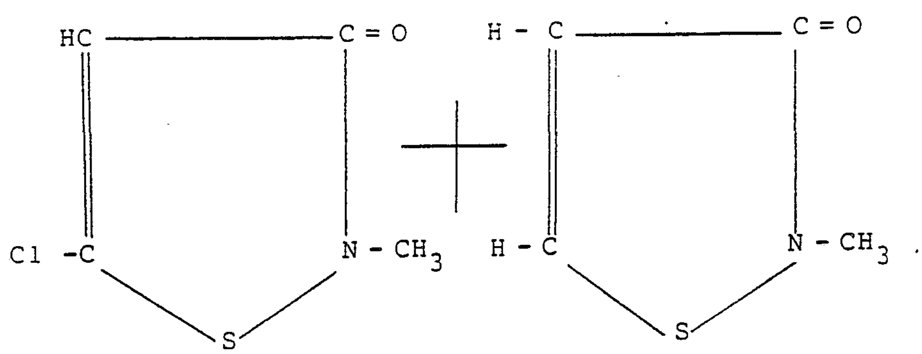

3. Utilisation suivant la revendication 1, caractérisée en ce que le composé d'ammonium quaternaire possède la formule

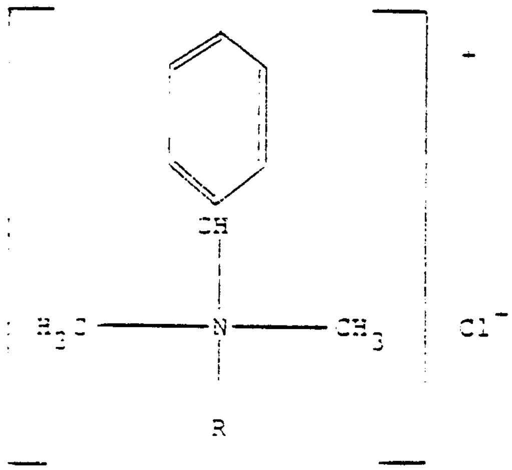

dans laquelle le substituant R est un alcoyle normal en $C_{12}$ à $C_{16}$.

4. Utilisation suivant la revendication 3, caractérisée ne ce que l'alcoyle normal se répartit en un alcoyle en $C_{12}$ pour 40 %, en un alcoyle en $C_{14}$ pour 50 % et en un alcoyle en $C_{16}$ pour 10 %.

5. Utilisation suivant la revendication 1 ou 2, caractérisée en ce que le rapport pondéral de la composition est de l'ordre de 9 : 1.

6. Utilisation de la composition suivant l'une des revendications 1 à 3, pour un produit fourrager en rapport pondéral d'une partie de la composition pour 50 à 250 parties du produit fourrager.

7. Utilisation de la composition suivant l'une des revendications 1 à 3, pour des denrées alimentaires dans une solution aqueuse en une concentration de 1 : 500 à 1 : 2 500.